# EUROPÄISCHE PATENTSCHRIFT

(11) **EP 2 815 707 B1**
(45) Veröffentlichungstag und Bekanntmachung des Hinweises auf die Patenterteilung: **10.04.2019**
(21) Anmeldenummer: 14172582.0
(22) Anmeldetag: 16.06.2014
(51) Int. Cl.: A61B 17/29, A61B 34/30

(54) **Endoskopisches Instrument**
Endoscopic instrument
Instrument endoscopique

(30) Priorität: 20.06.2013 DE 102013106446
(43) Veröffentlichungstag der Anmeldung: 24.12.2014
(73) Patentinhaber: Karl Storz SE & Co. KG, 78532 Tuttlingen (DE)
(72) Erfinder: Abri, Omid, 14129 Berlin (DE); Schrader, Stephan, 14532 Kleinmachnow (DE); Forster, Jonas, 13595 Berlin (DE)
(74) Vertreter: Witte, Weller & Partner Patentanwälte mbB

(56) Entgegenhaltungen:
- US-A1- 2002 143 358
- US-A1- 2002 156 497
- US-A1- 2004 253 079
- US-A1- 2006 229 666
- US-A1- 2012 296 356

## Beschreibung

Die vorliegende Erfindung betrifft ein endoskopisches Instrument.

Die klassischen Instrumente der minimal-invasiven Chirurgie (MIC) sind vom Griff (proximales Ende) bis hin zum Endeffektor (distales Ende), bei dem es sich insbesondere um einen Greifer oder eine Schere handelt, starr. Dabei stehen dem Chirurgen üblicherweise zwei Freiheitsgrade zur Verfügung. Zum einen kann der Schaft, und damit der Endeffektor, rotiert werden. Zum anderen kann der Endeffektor geöffnet und geschlossen werden.

Zwar ist es möglich, dass der Chirurg das endoskopische Instrument lateral verlagert, doch stellt dies nur eine geringe Bewegungsfreiheit dar. Diese Bewegungsfreiheit wird umso geringer, je tiefer ein Chirurg das endoskopische Instrument in den Körper eines Patienten einführen muss und je sensibler das umliegende Gewebe ist, das durch eine laterale Verlagerung des Instruments Schaden nehmen könnte.

Vor ähnliche Probleme sieht sich auch der Techniker gestellt, der mit einem endoskopischen Instrument in engen technischen Räumen, wie z.B. in einem Motor, arbeiten muss. Sowohl der Chirurg als auch der Techniker werden durch die geringe Manövrierfähigkeit eingeschränkt und müssen versuchen, über ein erhöhtes Maß an Geschick und Erfahrung diese Einschränkungen wettzumachen,

Im Stand der Technik sind daher verschiedene Lösungen vorgeschlagen worden, um die Beweglichkeit des endoskopischen Instruments an seinem distalen Ende zu verbessern.

US 6,699,235 zeigt ein endoskopisches Instrument, das am distalen Ende in zwei zueinander senkrecht stehenden Ebenen verschwenkt werden kann. Die Vorrichtung wird aber in erster Linie mit einem Endeffektor verwendet, der für seine Betätigung keinen eigenen Freiheitsgrad benötigt, wie z.B. ein Endeffektor zum Kauterisieren.

US 2008/0058861 zeigt ein endoskopisches Instrument mit einem beweglichen distalen Ende, an dem ein Greifer angeordnet ist. Der Aufbau dieses Instruments ist allerdings sehr komplex und gibt dem Chirurgen bei seiner Arbeit keine unmittelbare haptische Rückmeldung.

US 7,121,781 zeigt ein endoskopisches Instrument, dessen distales Ende schwenkbar auf einem Kugelgelenk gelagert ist. Drei Stifte ermöglichen es, das distale Greiferende um zwei Achsen zu verlagern und den Endeffektor zu öffnen und zu schließen. Die Betätigung des Endeffektors muss über eine maschinelle Ansteuerung erfolgen. Zudem fehlt es an einer unmittelbaren haptischen Rückmeldung an den Chirurgen.

US 2012/0296356 offenbart ein endoskopisches Instrument mit einem schwenkbaren Endeffektor, der aus einem - bezogen auf einen öffnungs- bzw. Schließvorgang - feststehenden ersten Effektorteil und einem schwenkbaren zweiten Endeffektorteil besteht. Mittels zweier Schubelemente wird der Endeffektor lateral verschwenkt, und mittels eines Schubelements wird der Endeffektor geöffnet und geschlossen.

US 2002/0156497 offenbart ein endoskopisches Instrument mit einem schwenkbaren Endeffektor, der ortsfest mit einem Schaft des Instruments gekoppelt ist. Mittels zweier Schubelemente wird der Endeffektor lateral verschwenkt, und mittels eines Schubelements wird der Endeffektor geöffnet und geschlossen. Sofern die Bewegungen aller drei Schubelemente koordiniert erfolgen, ist auch ein Verschwenken sekrecht zur lateralen Richtung möglich.

Es ist eine Aufgabe, ein verbessertes endoskopisches Instrument be-reitzustellen, das dem Chirurgen ausreichende Freiheit bei der Betätigung des Endeffektors gibt, während das endoskopische Instrument im Einsatz ist. Dabei soll das chirurgische Instrument insbesondere mechanisch einfach aufgebaut sein und eine direkte haptische Rückmeldung an den Chirurgen ermöglichen. Dabei soll möglichst auch eine intuitive Bedienung des endoskopischen Instruments gegeben sein, so dass die Bewegungen der Hand des Chirurgen unmittelbar auf den Endeffektor übertragen werden.

Nach einem Aspekt wird die Aufgabe gelöst durch ein endoskopisches Instrument mit:
- einem proximalen Ende,
- einem distalen Ende,
- einer Längsrichtung, entlang derer sich das endoskopische Instrument vom proximalen Ende zum distalen Ende erstreckt,
- einem Endeffektor am distalen Ende, der ein erstes Endeffektorteil und ein zweites Endeffektorteil aufweist,
- einem Verbindungselement am distalen Ende, an dem das erste und das zweite Endeffektorteil gelagert sind und das sich entlang einer Querrichtung erstreckt,
- einem ersten Schubelement,
- einem zweiten Schubelement,
- einem dritten Schubelement, und
- einem vierten Schubelement,
wobei sich die Schubelemente jeweils entlang der Längsrichtung erstrecken und entlang der Längsrichtung relativ zueinander verlagerbar sind, und wobei das erste Schubelement gelenkig am ersten Endeffektorteil gelagert ist, das zweite Schubelement gelenkig am zweiten Endeffektorteil gelagert ist, das dritte Schubelement gelenkig an dem Verbindungselement gelagert ist und das vierte Schubelement gelenkig an dem Verbindungselement gelagert ist.

Eine Besonderheit dieses endoskopischen Instruments ist darin zu sehen, dass sich mittels der vier Schubelemente, die insbesondere bis ins proximale Ende des endoskopischen Instruments geführt sind, viele Freiheitsgrade realisieren lassen. So ist es möglich, das erste und das zweite Endeffektorteil, die einen Endeffektor oder zumindest eine Baugruppe des Endeffektors darstellen, relativ zueinander zu verlagern, und zwar bezogen auf das Verbindungselement, an dem das erste und das zweite Endeffektorteil gelagert sind.

Des Weiteren ist es möglich, die beiden Endeffektorteile gleichsinnig und gleichmäßig zu verlagern, so dass der Endeffektor in einer ersten Ebene, die senkrecht zu der Querrichtung steht, entlang derer sich das Verbindungselement erstreckt, verschwenkt werden kann. Außerdem ist es möglich, den Endeffektor in einer zweiten Ebene zu verschwenken, die zu der ersten Ebene senkrecht steht und die durch die Längsrichtung die Querrichtung aufgespannt ist.

Eine gleichzeitige Verlagerung des ersten und des zweiten Endeffektorteils in der ersten Ebene und in der zweiten Ebene ist auch möglich. Dadurch kann der Endeffektor in alle Richtungen verschwenkt werden, insbesondere sind auch ovale oder kreisförmige Bewegungen des Endeffektors möglich. Das endoskopische Instrument kann weiterhin entlang seiner Längsrichtung verlagert werden und um eine Längsachse entlang der Längsrichtung gedreht werden.

Es sei an dieser Stelle darauf hingewiesen, dass die bislang eingeführten und später noch hinzutretenden Begriffe "Längsrichtung", "Längsachse", "Querrichtung", "Querachse", "Hochrichtung" und "Hochachse" lediglich einer besseren Orientierung und einem vereinfachten Verständnis des strukturellen Aufbaus dienen. Die Verwendung dieser Begriffe dient nicht dazu, eine bestimmte Beziehung zu einem anderen Bezugssystem, wie z.B. dem Schwerefeld der Erde, herzustellen.

Anhand der verschiedenen Begriffe soll vielmehr aufgezeigt werden, dass bestimmte Erstreckungen des endoskopischen Instruments und Bewegungen des Endeffektors in unterschiedliche Richtungen erfolgen können. Dabei stehen jeweils Längsrichtung und Querrichtung, Längsrichtung und Hochrichtung und Querrichtung und Hochrichtung in einem Winkel zueinander, zeigen also in unterschiedliche Raumrichtungen. Dabei lässt sich das endoskopische Instrument so ausrichten, dass Längsrichtung, Querrichtung und Hochrichtung jeweils paarweise in einem Winkel von mindestens 45°, bevorzugt von mindestens 75°, besonders bevorzugt von mindestens 85° und insbesondere zumindest in etwa senkrecht zueinanderstehen.

Insbesondere soll die Hochrichtung als senkrecht zur Querrichtung verstanden werden. Da das erste und das zweite Endeffektorteil relativ zueinander verschwenkbar sind, müssen eine erste Hochachse des ersten Endeffektorteils und eine zweite Hochachse des zweiten Endeffektorteils nicht in jeder Stellung des Endeffektors zusammenfallen oder parallel sein. Vielmehr wird sich bei der Betätigung des Endeffektorteils, insbesondere bei einem Öffnen und Schließen, ein Winkel zwischen der ersten und der zweiten Hochachse einstellen. Die erste und die zweite Hochachse bleiben aber, abgesehen von extremen Stellungen des Endeffektorteils, jeweils in einem Winkel sowohl zur Querrichtung als auch zur Längsrichtung und sind in ihrer Ausrichtung daher technisch von der Längsrichtung der Querrichtung unterscheidbar.

Die Orientierungshilfe, die mit diesen Bezeichnungen erzielt werden soll, wird in den Figuren nochmals verdeutlicht.

Die Schubelemente erstrecken sich jeweils entlang der Längsrichtung, also entlang der Richtung, in der sich das endoskopische Instrument vom proximalen Ende zum distalen Ende erstreckt.

Es sei darauf hingewiesen, dass die Bezeichnungen "Schubelement", "Schubstange", "Schubkraft", sowohl im Sinne eines positiven Schubs als auch eines negativen Schubs, auch Zug genannt, zu verstehen sind. Die Begriffe werden lediglich als sprachliche Vereinfachung für ein besseres Verständnis der Erfindung verwendet.

Bevorzugt ist die Kopplung zwischen dem ersten Schubelement, dem ersten Endeffektorteil, dem zweiten Schubelement und dem zweiten Endeffektorteil derart ausgestaltet, dass eine Verlagerung des ersten Schubelements und des zweiten Schubelements auf das distale Ende zu das erste und das zweite Endeffektorteil in Schließrichtung aufeinander zubewegt und dass eine Verlagerung des ersten Schubelements und des zweiten Schubelements auf das proximale Ende zu das erste und das zweite Endeffektorteil in Öffnungsrichtung voneinander wegbewegt. Insbesondere wird diese Kopplung unabhängig von einer Bewegung des dritten und vierten Schubelements ausgestaltet, so dass die Kopplung bevorzugt ohne eine Verlagerung des dritten und vierten Schubelements erfolgt und das dritte und vierte Schubelement durch die Verlagerung von erstem und zweitem Schubelement nicht zwangsläufig verlagert werden.

Bevorzugt ist die Kopplung zwischen dem ersten Schubelement, dem ersten Endeffektorteil, dem zweiten Schubelement und dem zweiten Endeffektorteil derart ausgestaltet, dass eine gegenläufige Verlagerung des ersten Schubelements und des zweiten Schubelements bezogen auf die Längsrichtung ein Verschwenken von erstem und zweitem Endeffektorteil um das Verbindungselement bewirkt. Insbesondere wird diese Kopplung unabhängig von einer Bewegung des dritten und vierten Schubelements ausgestaltet, so dass die Kopplung bevorzugt ohne eine Verlagerung des dritten und vierten Schubelements erfolgt und das dritte und vierte Schubelement durch die Verlagerung von erstem und zweitem Schubelement nicht zwangsläufig verlagert werden.

Bevorzugt ist die Kopplung zwischen dem dritten Schubelement, dem vierten Schubelement und dem Verbindungselement derart ausgestaltet, dass eine gegenläufige Verlagerung des dritten Schubelements und des vierten Schubelements bezogen auf die Längsrichtung ein gemeinsames Verschwenken von erstem und zweitem Endeffektorteil jeweils um eine dritte und eine vierte Hochachse bewirkt, wobei die dritte Hochachse und vierte Hochachse jeweils senkrecht zur Querrichtung stehen. Wenn dritte und vierte Hochachse zusammenfallen, so kann dies bevorzugt die Hochrichtung definieren, entlang derer die dritte und vierte Hochachse verlaufen.

Damit ist die Aufgabe vollständig gelöst.

Bei einer vorteilhaften Ausgestaltung ist das erste Schubelement mittels eines ersten Gelenks gelenkig am ersten Endeffektorteil gelagert und/oder ist das zweite Schubelement mittels eines zweiten Gelenks gelenkig am zweiten Endeffektorteil gelagert.

Diese Ausgestaltung ermöglicht eine gute Übertragung der Schub- bzw. Zugkraft von den Schubelementen auf die Endeffektorteile. Außerdem können die Endeffektorteile präzise verlagert werden. Es ist vorteilhaft, wenn beide gelenkige Lagerungen jeweils als Gelenk ausgeführt sind. Alternativ kann statt eines Gelenks auch ein elastisches Bauteil, z.B. aus Gummi oder Kunststoff, verwendet werden.

Bei einer vorteilhaften Ausgestaltung der Erfindung ist die gelenkige Lagerung des ersten Schubelements derart ausgebildet, dass das erste Endeffektorteil relativ zu dem ersten Schubelement parallel zu einer Schwenkebene verlagerbar ist und um eine Querachse des Verbindungselements verschwenkt werden kann, wobei die Schwenkebene eine gedachte Ebene ist, die durch die Längsrichtung und die Querrichtung aufgespannt ist.

Diese Art der gelenkigen Lagerung ermöglicht besonders viele Freiheitsgrade bei dem Verschwenken und Betätigen des Endeffektors.

Alternativ oder zusätzlich ist es vorteilhaft, wenn die gelenkige Lagerung des zweiten Schubelements derart ausgebildet ist, dass das zweite Endeffektorteil relativ zu dem zweiten Schubelement parallel zu einer Schwenkebene verlagerbar ist und um eine Querachse des Verbindungselements verschwenkt werden kann, wobei die Schwenkebene wiederum eine gedachte Ebene ist, die durch die Längsrichtung und die Querrichtung aufgespannt ist.

Bei einer weiteren vorteilhaften Ausgestaltung der Erfindung ist die gelenkige Lagerung des dritten Schubelements derart ausgebildet, dass das dritte Schubelement und das Verbindungselement um eine Schwenkachse relativ zueinander verschwenkbar sind.

Diese Ausgestaltung ermöglicht eine besonders gute Verschwenkbarkeit des Endeffektors. Dabei ist es insbesondere vorteilhaft, wenn die gelenkige Lagerung derart ausgestaltet ist, dass ein Verschwenken ausschließlich um die dritte Hochachse möglich ist. Wie bereits erläutert, kann die dritte Hochachse entlang der Hochrichtung verlaufen. In verschiedenen Bedienzuständen des Endeffektorteils wird die dritte Hochachse aber üblicherweise in einem Winkel zur Hochrichtung stehen.

Alternativ oder zusätzlich ist es bevorzugt, wenn die gelenkige Lagerung des vierten Schubelements derart ausgebildet ist, dass das vierte Schubelement und das Verbindungselement um die Schwenkachse relativ zueinander verschwenkbar sind.

Bei einer bevorzugten Ausgestaltung ist die gelenkige Lagerung des dritten Schubelements derart ausgebildet, dass ein Vorsprung am dritten Schubelement in eine Ausnehmung des Verbindungselements greift oder dass ein Vorsprung am Verbindungselement in eine Ausnehmung des dritten Schubelements greift oder ein dritter Bolzen in zwei Ausnehmungen des Verbindungselements eingesetzt ist und dazwischen durch eine Ausnehmung am dritten Schubelement geführt ist.

Diese Ausgestaltung bietet eine konstruktiv einfache Möglichkeit der gelenkigen Lagerung. Dabei ist es vorteilhaft, wenn zwei Vorsprünge am dritten Schubelement entlang einer dritten Hochachse in Ausnehmungen des Verbindungselements greifen oder zwei Vorsprünge am Verbindungselement in eine oder mehrere Ausnehmungen des dritten Schubelements greifen.

Alternativ oder zusätzlich ist es vorteilhaft, wenn die gelenkige Lagerung des vierten Schubelements derart ausgestaltet ist, dass ein Vorsprung am vierten Schubelement in eine Ausnehmung des Verbindungselements greift oder dass ein Vorsprung am Verbindungselement in eine Ausnehmung des vierten Schubelements greift oder ein vierter Bolzen in zwei Ausnehmungen des Verbindungselements eingesetzt ist und dazwischen durch eine Ausnehmung am vierten Schubelement geführt ist.

Bei einer vorteilhaften Ausgestaltung der Erfindung verläuft eine gedachte Verlängerung einer ersten Hochachse, um die das erste Endeffektorteil relativ zum ersten Schubelement verschwenkbar ist, bezogen auf die Querrichtung zwischen Anlenkpunkten des dritten und vierten Schubelements an dem Verbindungselement, insbesondere in der Mitte zwischen den Anlenkpunkten.

Diese Ausgestaltung ermöglicht eine besonders gute Steuerbarkeit des Endeffektors. Bei alternativen bevorzugten Ausgestaltungen verläuft die gedachte Verlängerung der ersten Hochachse außerhalb der Mitte zwischen den Anlenkpunkten. Es ist zudem vorteilhaft, wenn die gedachte Verlängerung der ersten Hochachse durch den Anlenkpunkt des dritten Schubelements oder den Anlenkpunkt des vierten Schubelements verläuft.

Bei einer weiteren vorteilhaften Ausgestaltung der Erfindung ist eine erste Querachse, um die das erste Endeffektorteil relativ zum ersten Schubelement verschwenkbar ist, zumindest in etwa parallel zur Querrichtung.

Diese Ausgestaltung ist mechanisch vorteilhaft und erlaubt eine besonders präzise Steuerung des Endeffektors. Mit entsprechenden technischen Maßnahmen ist es aber auch möglich, die erste Querachse in einem Winkel zur Querrichtung zu wählen.

Alternativ oder zusätzlich ist es bevorzugt, wenn eine zweite Querachse, um die das zweite Endeffektorteil relativ zum zweiten Schubelement verschwenkbar ist, zumindest in etwa parallel zur Querrichtung ist.

Bei einer vorteilhaften Ausgestaltung weist das erste Gelenk eine erste Gelenkhülse auf, die drehbar in eine Ausnehmung an dem ersten Endeffektorteil eingesetzt ist.

Diese Ausgestaltung ermöglicht eine besonders stabile und gute Führung, die besonders gut für eine Aufnahme von Schubkräften ausgebildet sein kann. Hierzu liegt die Gelenkhülse bevorzugt flächig an der Ausnehmung an. Insbesondere ist es bevorzugt, die Gelenkhülse in eine zumindest in etwa zylinderförmige Ausnehmung einzusetzen. Alternativ oder zusätzlich ist es bevorzugt, wenn das zweite Gelenk eine zweite Gelenkhülse aufweist, die drehbar in eine zweite Ausnehmung an dem zweiten Endeffektorteil eingesetzt ist.

Bei einer bevorzugten Ausgestaltung ist das erste Schubelement formschlüssig mit der ersten Gelenkhülse verbunden.

Diese Ausgestaltung der Verbindung kann einfach realisiert werden und ist zuverlässig. Als Alternativen für die formschlüssige Verbindung stehen dabei bevorzugt dieselben Alternativen zur Verfügung, wie sie zuvor im Zusammenhang mit der gelenkigen Lagerung des dritten Schubelements am Verbindungselement beschrieben wurden. Alternativ oder zusätzlich ist das zweite Schubelement formschlüssig mit der zweiten Gelenkhülse verbunden.

Bei einer weiteren vorteilhaften Ausgestaltung ist die erste Gelenkhülse nur um eine erste Hochachse drehbar.

Diese Ausgestaltung ermöglicht eine präzise Steuerung des Endeffektors, da die Gelenkhülse genau einen Freiheitsgrad, nämlich eine Verlagerung um die erste Hochachse, bereitstellt. Alternativ oder zusätzlich ist es bevorzugt, wenn die zweite Gelenkhülse nur um eine zweite Hochachse drehbar ist, wobei die zweite Hochachse, insbesondere in einer gedachten Verlängerung mit der ersten Hochachse, zusammenfällt.

Bei einer weiteren vorteilhaften Ausgestaltung der Erfindung ist das erste Schubelement relativ zur ersten Gelenkhülse nur in einer gedachten Ebene verlagerbar, die durch die Längsrichtung und die Hochrichtung aufgespannt ist.

Auch diese Ausgestaltung ermöglicht eine präzise Steuerung des Endeffektors. Wie bereits ausgeführt, ist die Hochrichtung sowohl von der Längsrichtung als auch von der Querrichtung verschieden und steht insbesondere senkrecht zur Querrichtung. Alternativ oder zusätzlich ist es bevorzugt, wenn das zweite Schubelement relativ zur zweiten Gelenkhülse nur in einer zweiten gedachten Ebene verlagerbar ist, die durch die Längsrichtung und eine Hochrichtung aufgespannt ist.

Bei einer weiteren vorteilhaften Ausgestaltung der Erfindung sind alle Schubelemente parallel zueinander angeordnet.

Diese Ausgestaltung ist konstruktiv einfach und zuverlässig. Außerdem kann der Durchmesser des endoskopischen Instruments dadurch gering gehalten werden.

Bei einer weiteren vorteilhaften Ausgestaltung der Erfindung sind die Schubelemente als Schubstangen ausgestaltet, insbesondere vollständig geradlinig ausgestaltet.

Auch diese Ausgestaltung ist konstruktiv einfach und zuverlässig.

Bei einer weiteren vorteilhaften Ausgestaltung der Erfindung ist das erste Endeffektorteil als erstes Greifzangenteil und das zweite Endeffektorteil als zweites Greifzangenteil ausgebildet oder ist das erste Endeffektorteil als erstes Scherenteil und das zweite Endeffektorteil als zweites Scherenteil ausgebildet.

Diese Ausgestaltungen sind besonders vorteilhaft, da dem Benutzer auf einfache Weise alle erforderlichen Freiheitsgrade zur Verfügung gestellt werden können. Grundsätzlich können aber auch andere Endeffektoren verwendet werden, wie z.B. Zangen, Scheren, Koagulationsnadeln oder Fluidabsaugungen, sowie Maulteile.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein erstes Ausführungsbeispiel eines endoskopischen Instruments in perspektivischer Ansicht;
- Fig. 2: das erste Ausführungsbeispiel in der Draufsicht;
- Fig. 3: das erste Ausführungsbeispiel in einer seitlichen Ansicht;
- Fig. 4: das erste Ausführungsbeispiel in einer Explosionsansicht;
- Fig. 5: die Schubstangen des ersten Ausführungsbeispiels;
- Fig. 6: die Darstellung gemäß Fig. 5 zusätzlich mit dem Verbindungselement;
- Fig. 7: die Darstellung gemäß Fig. 6 zusätzlich mit Gelenkhülsen;
- Fig. 8: die Darstellung gemäß Fig. 7 zusätzlich mit dem zweiten Endeffektorteil;
- Fig. 9: die Darstellung gemäß Fig. 8 zusätzlich mit dem ersten Endeffektorteil;
- Fig. 10: die Darstellung gemäß Fig. 9 zusätzlich mit Abdeckkappen zum Halten der Gelenkhülsen;
- Fig. 11: ein zweites Ausführungsbeispiel in perspektivischer Ansicht;
- Fig. 12: eine Vergrößerung des distalen Endes des zweiten Ausführungsbeispiels aus einer ersten Perspektive; und
- Fig. 13: eine Vergrößerung des distalen Endes des zweiten Ausführungsbeispiels aus einer zweiten Perspektive.

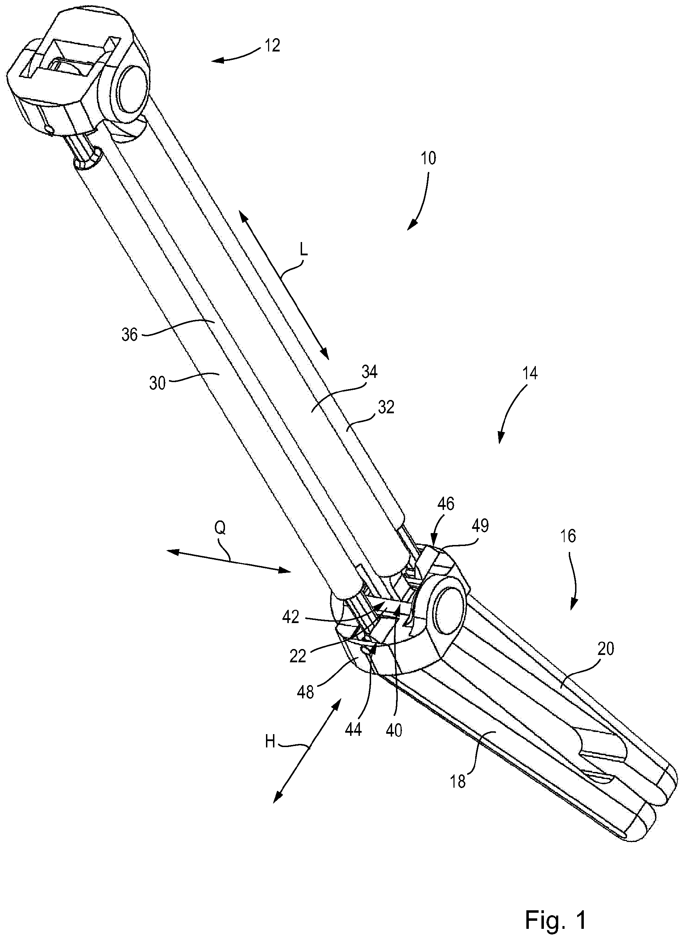

Fig. 1 zeigt ein erstes Ausführungsbeispiel eines endoskopischen Instruments 10 mit einem proximalen Ende 12 und einem distalen Ende 14. Als Orientierung sind eine Längsrichtung L, entlang derer sich das endoskopische Instrument 10 vom proximalen Ende 12 zum distalen Ende 14 erstreckt, eine Querrichtung Q, die quer zur Längsrichtung L verläuft, und eine Hochrichtung H eingezeichnet, wobei die Hochrichtung H sowohl quer zur Längsrichtung L als auch quer zur Querrichtung Q verläuft.

Am distalen Ende 14 weist das endoskopische Instrument 10 einen Endeffektor 16 auf, der ein erstes Endeffektorteil 18 und ein zweites Endeffektorteil 20 aufweist. Ferner ist am distalen Ende 14 ein Verbindungselement 22 angeordnet, an dem das erste und das zweite Endeffektorteil 18, 20 gelagert sind und das sich entlang der Querrichtung Q erstreckt.

Das Instrument 10 weist ein erstes Schubelement 30, ein zweites Schubelement 32, ein drittes Schubelement 34 und ein viertes Schubelement 36 auf. Die Schubelemente 30, 32, 34, 36 erstrecken sich jeweils entlang der Längsrichtung L und sind entlang der Längsrichtung L relativ zueinander verlagerbar.

Das erste Schubelement 30 ist gelenkig am ersten Endeffektorteil 18 gelagert. Das zweite Schubelement 32 ist gelenkig am zweiten Endeffektorteil 20 gelagert. Das dritte Schubelement 34 ist gelenkig an dem Verbindungselement 22 gelagert, und zwar an einem dritten Anlenkpunkt 40, der hier verdeckt ist. Das vierte Schubelement 36 ist gelenkig an dem Verbindungselement 22 gelagert, und zwar an einem vierten Anlenkpunkt 42, der hier verdeckt ist.

Das erste Schubelement 30 ist mittels eines ersten Gelenks 44 gelenkig am ersten Endeffektorteil 18 gelagert. Außerdem ist auch das zweite Schubelement 32 mittels eines zweiten Gelenks 46 gelenkig am zweiten Endeffektorteil 20 gelagert. Es ist zwar grundsätzlich möglich, zumindest eines der Schubelemente 30, 32 starr mit dem jeweiligen Endeffektorteil 18, 20 zu koppeln, doch wird ein besonderer Vorteil darin gesehen, dass beide Endeffektorteile 18, 20 individuell bewegt werden können.

Ferner sind ein erstes Halteelement 48 und ein zweites Halteelement 49 gezeigt, deren Funktion noch im weiteren Verlauf erläutert wird.

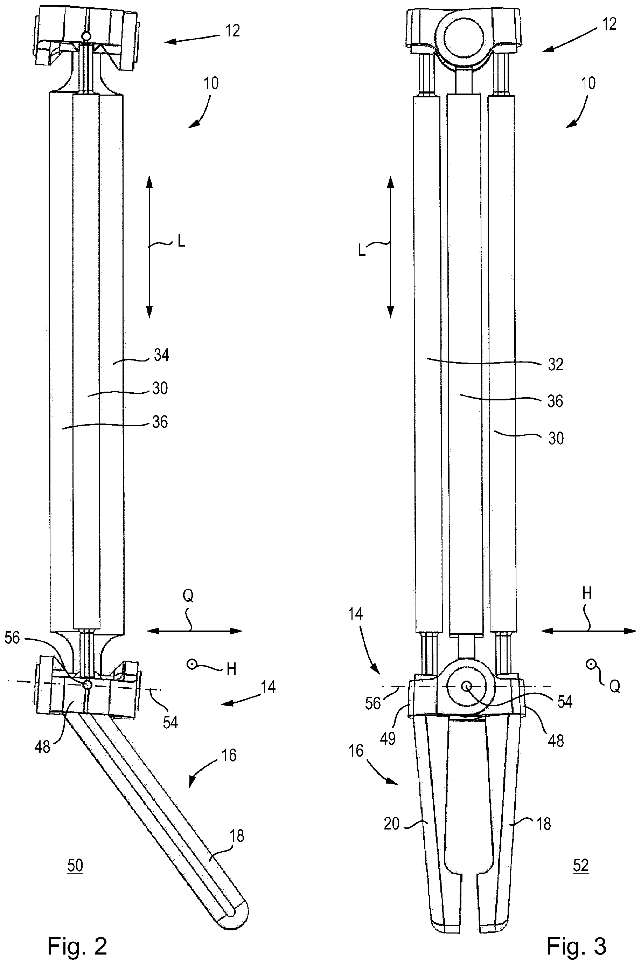

In Fig. 2 ist das endoskopische Instrument 10 gemäß Fig. 1 in einer Draufsicht gezeigt. Dabei ist die Zeichenebene die Ebene, die durch die Längsrichtung L und die Querrichtung Q aufgespannt ist. Diese Ebene soll als Schwenkebene 50 verstanden werden. Die Hochrichtung H steht hier senkrecht zu der Schwenkebene 50.

Fig. 3 zeigt das endoskopische Instrument 10 gemäß Fig. 1 in einer Seitenansicht. Die Zeichenebene ist die Ebene, die von der Längsrichtung L und der Hochrichtung H aufgespannt ist und ist mit dem Bezugszeichen 52 gekennzeichnet.

Die gelenkige Lagerung des Schubelements 30 ist derart ausgebildet, dass das erste Endeffektorteil 18 relativ zu dem ersten Schubelement 30 parallel zu einer Schwenkebene 50 verlagerbar ist und um eine Querachse 54 des Verbindungselements 22 verschwenkt werden kann. Wie bereits erläutert, ist die Schwenkebene 50 eine gedachte Ebene, die durch die Längsrichtung L und die Querrichtung Q aufgespannt ist.

Die gelenkige Lagerung des dritten Schubelements 34 ist derart ausgebildet, dass das dritte Schubelement 34 und das Verbindungselement 22 um eine Schwenkachse 56 relativ zueinander verschwenkbar sind. In der Draufsicht gemäß Fig. 2 steht die Schwenkachse 56 senkrecht zur Zeichenebene. Bei der Seitenansicht gemäß Fig. 3 liegt die Schwenkachse 56 in der Zeichenebene. Die Querachse 54 des Verbindungselements schneidet hier die Schwenkachse 56. Die gelenkige Lagerung des vierten Schubelements 36 ist derart ausgebildet, dass das vierte Schubelement 36 und das Verbindungselement 22 um die Schwenkachse 56 relativ zueinander verschwenkbar sind.

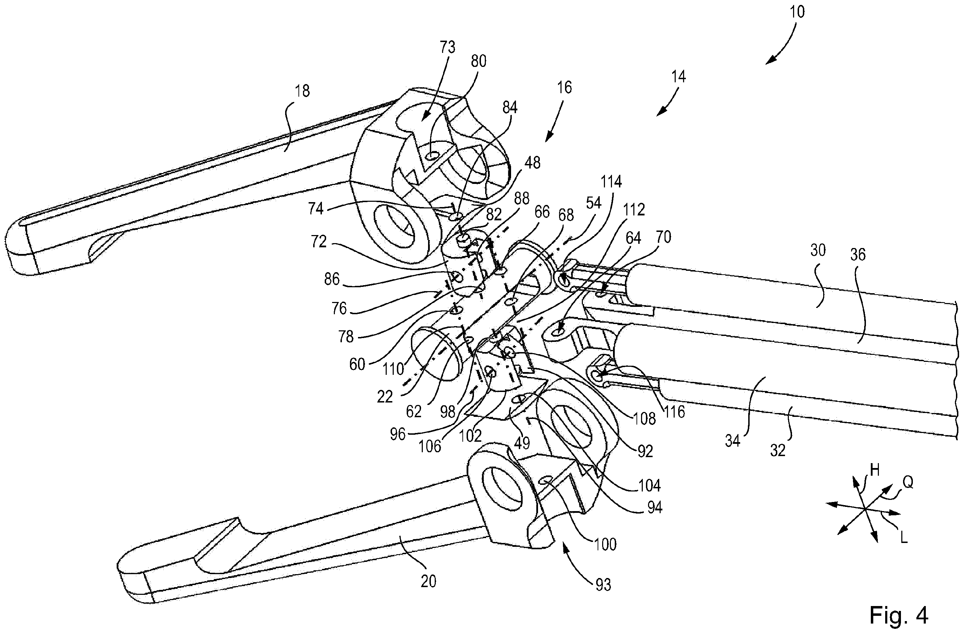

Fig. 4 zeigt eine Explosionsansicht des Endeffektors 16, wobei das erste Endeffektorteil 18 und das zweite Endeffektorteil 20 für eine bessere Übersichtlichkeit besonders weit nach außen verschoben wurden.

Die gelenkige Lagerung des dritten Schubelements 34 ist derart ausgebildet, dass ein dritter Bolzen (nicht gezeigt) in eine dritte Ausnehmung 60, des Verbindungselements 22 und in eine weitere dritte Ausnehmung 62 des Verbindungselements 22 eingesetzt ist und dazwischen durch eine dritte Ausnehmung 64 am dritten Schubelement 34 geführt ist. Alternativ dazu kann die gelenkige Lagerung des dritten Schubelements 34 derart ausgebildet sein, dass ein Vorsprung am dritten Schubelement 34 in eine Ausnehmung 60; 62 des Verbindungselements 22 greift oder dass ein Vorsprung am Verbindungselement in die dritte Ausnehmung 64 des dritten Schubelements 34 greift.

Die gelenkige Lagerung des vierten Schubelements 36 ist derart ausgebildet, dass ein vierter Bolzen (nicht gezeigt) in eine vierte Ausnehmung 66 des Verbindungselements 22 und in eine weitere vierte Ausnehmung 68 des Verbindungselements 22 eingesetzt ist und dazwischen durch eine vierte Ausnehmung 70 am vierten Schubelement 36 geführt ist. Alternativ dazu kann die gelenkige Lagerung des vierten Schubelements 36 derart ausgebildet sein, dass ein Vorsprung am vierten Schubelement 36 in eine Ausnehmung 66; 68 des Verbindungselements 22 greift oder dass ein Vorsprung am Verbindungselement in die vierte Ausnehmung 70 des vierten Schubelements 36 greift.

Das erste Gelenk 44 weist eine erste Gelenkhülse 72 auf, die drehbar in eine erste Ausnehmung 73 an dem ersten Endeffektorteil 18 eingesetzt ist. Die erste Gelenkhülse 72 hat eine erste Hochachse 74, um die sich das erste Endeffektorteil 18 relativ zur ersten Gelenkhülse 72 verlagern kann. Die erste Gelenkhülse 72 hat eine erste Querachse 76, um die sich das erste Schubelement 30 relativ zur ersten Gelenkhülse 72 verlagern kann.

Die erste Gelenkhülse 72 ist mit einem ersten Vorsprung 78 in eine erste Aufnahme 80 eingesetzt und so formschlüssig mit dem ersten Endeffektorteil 18 verbunden. Die erste Gelenkhülse 72 ist ferner über einen weiteren ersten Vorsprung 82 in eine erste Aufnahme 84 des ersten Halteelements 48 eingesetzt. Die erste Gelenkhülse 72 weist außerdem eine erste Ausnehmung 86 und eine weitere erste Ausnehmung 88 auf, durch die die erste Querachse 76 verläuft.

Das zweite Gelenk 46 weist eine zweite Gelenkhülse 92 auf, die drehbar in eine zweite Ausnehmung 93 an dem zweiten Endeffektorteil 20 eingesetzt ist. Die zweite Gelenkhülse 92 hat eine zweite Hochachse 94, um die sich das zweite Endeffektorteil 20 relativ zur zweiten Gelenkhülse 92 verlagern kann. Die zweite Gelenkhülse 92 hat eine zweite Querachse 96, um die sich das zweite Schubelement 32 relativ zur zweiten Gelenkhülse 92 verlagern kann.

Die zweite Gelenkhülse 92 ist mit einem zweiten Vorsprung 98 in eine zweite Aufnahme 100 eingesetzt und so formschlüssig mit dem zweiten Endeffektorteil 20 verbunden. Die zweite Gelenkhülse 92 ist ferner über einen weiteren zweiten Vorsprung 102 (verdeckt) in eine zweite Aufnahme 104 des zweiten Halteelements 49 eingesetzt. Die zweite Gelenkhülse 92 weist außerdem eine zweite Ausnehmung 106 und eine weitere zweite Ausnehmung 108 auf, durch die die zweite Querachse 96 verläuft.

Ferner sind in der Fig. 4 die dritte Hochachse 110 und die vierte Hochachse 112 eingezeichnet, um die sich das dritte Schubelement 34 bzw. das vierte Schubelement 36 verlagern können.

Das erste Schubelement 30 ist formschlüssig mit der ersten Gelenkhülse 72 verbunden. Bei dieser konkreten Ausgestaltung ist die formschlüssige Verbindung dadurch realisiert, dass ein erster Bolzen (nicht gezeigt) in die erste Ausnehmung 86 und die weitere erste Ausnehmung 88 eingesetzt ist und dazwischen durch eine erste Ausnehmung 114 am ersten Schubelement 30 geführt ist.

Das zweite Schubelement 32 ist formschlüssig mit der zweiten Gelenkhülse 92 verbunden. Bei dieser konkreten Ausgestaltung ist die formschlüssige Verbindung dadurch realisiert, dass ein zweiter Bolzen (nicht gezeigt) in die zweite Ausnehmung 106 und die weitere zweite Ausnehmung 108 eingesetzt ist und dazwischen durch eine zweite Ausnehmung 116 am zweiten Schubelement 32 geführt ist.

Die erste Gelenkhülse 72 ist hier nur um die erste Hochachse 74 drehbar. Das erste Schubelement 30 ist relativ zur ersten Gelenkhülse 72 nur in einer gedachten Ebene verlagerbar, die durch die Längsrichtung L und die Hochrichtung H aufgespannt ist.

Die zweite Gelenkhülse 74 ist hier nur um die zweite Hochachse 94 drehbar. Das zweite Schubelement 32 ist relativ zur zweiten Gelenkhülse 92 nur in einer gedachten Ebene verlagerbar, die durch die Längsrichtung L und die Hochrichtung H aufgespannt ist.

Eine gedachte Verlängerung der ersten Hochachse 74, um die das erste Endeffektorteil 18 relativ zum ersten Schubelement 30 verschwenkbar ist, verläuft bezogen auf die Querrichtung Q in der Mitte zwischen den Anlenkpunkten 40, 42 des dritten und vierten Schubelements 34, 36 an dem Verbindungselement 22.

Eine gedachte Verlängerung der zweiten Hochachse 94, um die das zweite Endeffektorteil 20 relativ zum zweiten Schubelement 32 verschwenkbar ist, verläuft bezogen auf die Querrichtung Q ebenfalls in der Mitte zwischen den Anlenkpunkten 40, 42 des dritten und vierten Schubelements 34, 36 an dem Verbindungselement 22.

Alle Schubelemente 30, 32, 34, 36 sind parallel zueinander angeordnet. Die Schubelemente 30, 32, 34, 36 sind hier als Schubstangen ausgestaltet und dabei insbesondere vollständig geradlinig ausgestaltet.

Bei der hier gezeigten Ausführungsform ist das erste Endeffektorteil 18 als erstes Greifzangenteil und das zweite Endeffektorteil 20 als zweites Greifzangenteil ausgebildet. Alternativ kann das erste Endeffektorteil bevorzugt als erstes Scherenteil und das zweite Endeffektorteil 20 als zweites Scherenteil ausgebildet werden. Selbstverständlich können auch andere Endeffektorteile verwendet werden.

In den nachfolgenden Figuren wird nun schrittweise der Aufbau und ein Teil des Zusammenbaus des endoskopischen Instruments 10 erläutert.

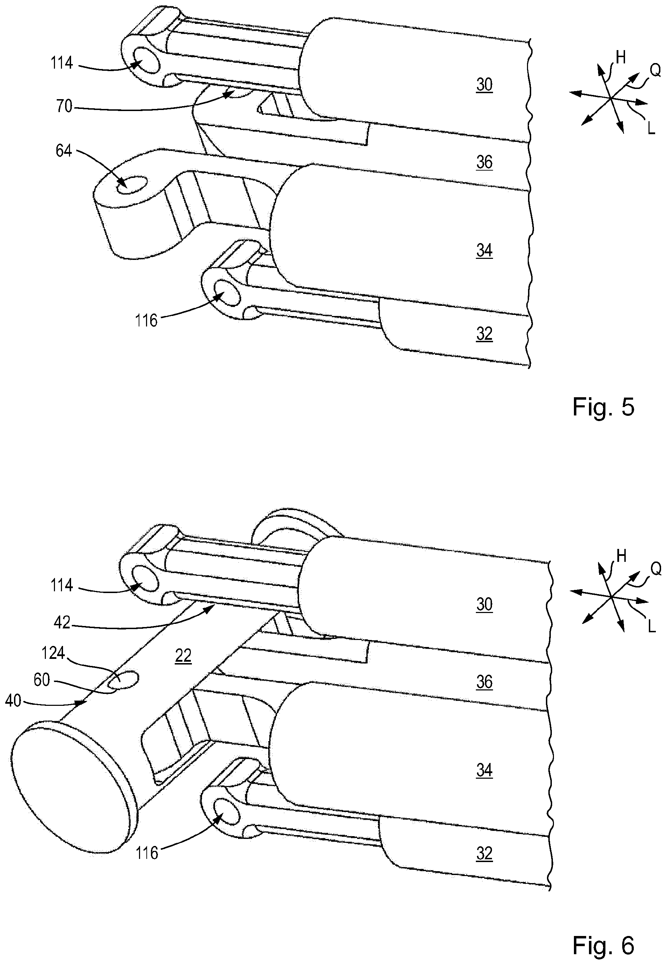

Fig. 5 zeigt die vier Schubelemente 30, 32, 34, 36 mit ihren entsprechenden Ausnehmungen 114, 116, 64, 70.

Fig. 6 zeigt, wie das dritte und vierte Schubelement 34, 36 mit dem Verbindungselement 22 gekoppelt werden. Das dritte Schubelement 34 ist mit einem dritten Bolzen 124 mit dem Verbindungselement 22 gekoppelt, der in die Ausnehmung 60 und in die gegenüberliegende Ausnehmung 62 (hier verdeckt) des Verbindungselements 22 eingesetzt ist. Hieraus resultiert ein dritter Anlenkpunkt 40. Das vierte Schubelement 36 ist mit einem vierten Bolzen (hier verdeckt) mit dem Verbindungselement 22 gekoppelt, der in die Ausnehmung 64 (hier verdeckt) und in die gegenüberliegende Ausnehmung 66 (hier verdeckt) des Verbindungselements 22 eingesetzt ist. Hieraus resultiert ein vierter Anlenkpunkt 42 (hier verdeckt).

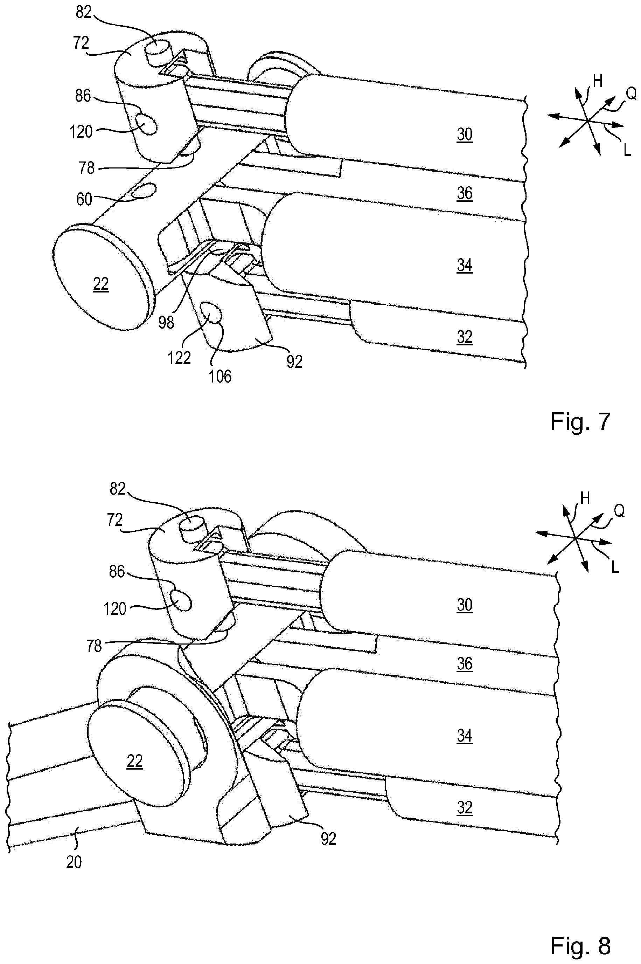

Fig. 7 zeigt zusätzlich, wie das erste Schubelement 30 mit einem ersten Bolzen 120 mit der ersten Gelenkhülse 72 gekoppelt ist. Es ist außerdem gezeigt, wie das zweite Schubelement 32 mit einem zweiten Bolzen 122 mit der zweiten Gelenkhülse 92 gekoppelt ist. Es ist hier zu erkennen, dass die Gelenkhülsen 72, 92 jeweils eine Abschrägung aufweisen. Da die Gelenkhülsen 72, 92 aufgrund ihrer Kopplung mit den Endeffektorteilen 18, 20 ebenfalls um die Querachse 54 des Verbindungselements 22 verlagert werden, wird so ein Endanschlag bei der Öffnung des Endeffektorteils 16 realisiert.

Fig. 8 zeigt zusätzlich das zweite Endeffektorteil 20. Die zweite Gelenkhülse 92 ist in die zweite Ausnehmung 93 eingesetzt.

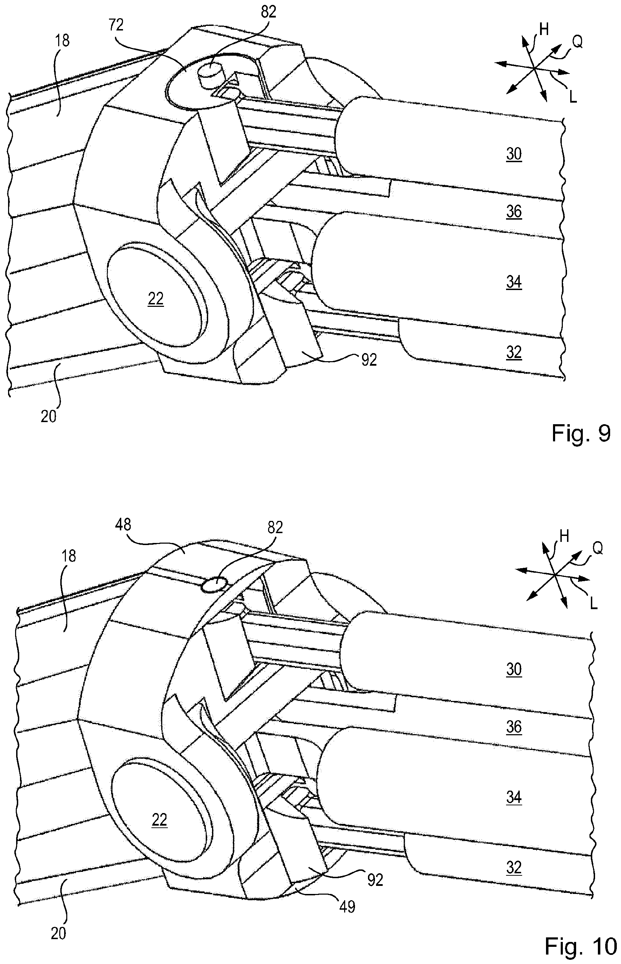

Fig. 9 zeigt zusätzlich das erste Endeffektorteil 18. Die erste Gelenkhülse 72 ist in die erste Ausnehmung 73 eingesetzt.

Fig. 10 zeigt schließlich zusätzlich die beiden Halteelemente 48, 49, die die erste und zweite Gelenkhülse 72, 92 fixieren.

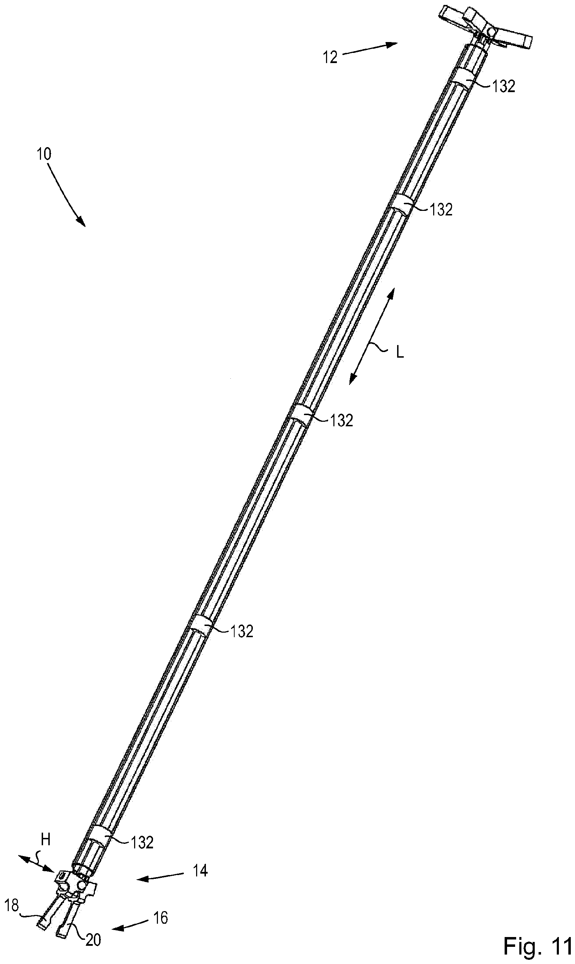

Fig. 11 zeigt ein zweites Ausführungsbeispiel eines endoskopischen Instruments 10. Dabei werden die Elemente, die eine funktionale Entsprechung oder funktionale Ähnlichkeit zu den bisher eingeführten Elementen haben, ebenfalls mit den bisher eingeführten Bezugszeichen bezeichnet. Diese bereits eingeführten Elemente werden nachfolgend nicht erneut erläutert.

In Fig. 11 ist gut zu erkennen, dass die Erfindung insbesondere auch für besonders dünne endoskopische Instrumente vorteilhaft ist, die relativ zu ihrem Querschnitt eine vielfach höhere Längserstreckung haben.

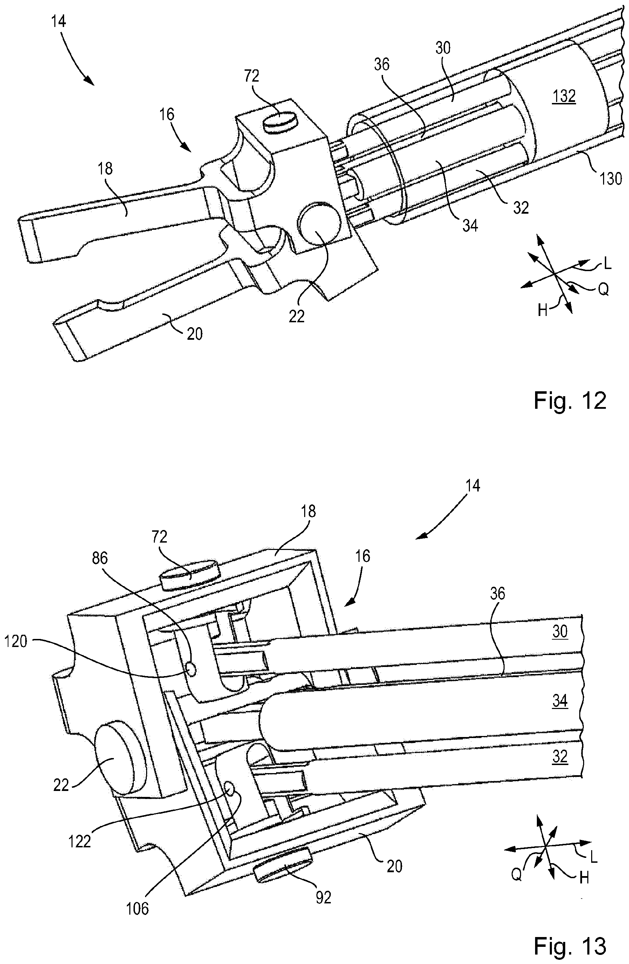

Fig. 12 zeigt, dass bei dieser Ausführungsform die Schubelemente 30, 32, 34, 36 in einem Schaft 130 angeordnet sind, der hier für ein besseres Verständnis transparent dargestellt ist. Die Führung der Schubelemente 30, 32, 34, 36 wird in vorteilhafter Weise durch Führungselemente 132 gewährleistet, die in dem Schaft 130 angeordnet sind und durch die mindestens zwei, bevorzugt alle Schubelemente 30, 32, 34, 36 geführt sind.

Fig. 13 verdeutlicht, dass bei der zweiten Ausführungsform auch eine alternative Ausgestaltung der Gelenkhülsen 72, 92 gewählt wurde.

Damit wurde insgesamt ein besonders vorteilhaftes endoskopisches Instrument 10 aufgezeigt, das mit einer mechanisch zu realisierenden Lösung dem Chirurgen viele Freiheitsgrade für die Betätigung des Endeffektors bietet und dabei auch eine intuitive Bedienbarkeit ermöglicht.

## Patentansprüche

1. Endoskopisches Instrument (10) mit
- einem proximalen Ende (12),
- einem distalen Ende (14),
- einer Längsrichtung (L), entlang derer sich das endoskopische Instrument (10) vom proximalen Ende (12) zum distalen Ende (14) erstreckt,
- einem Endeffektor (16) am distalen Ende (14), der ein erstes Endeffektorteil (18) und ein zweites Endeffektorteil (20) aufweist,
- einem Verbindungselement (22) am distalen Ende (14), an dem das erste und das zweite Endeffektorteil (18, 20) gelagert sind und das sich entlang einer Querrichtung (Q) erstreckt,
- einem ersten Schubelement (30),
- einem zweiten Schubelement (32),
- einem dritten Schubelement (34), und
- einem vierten Schubelement (36),
wobei sich die Schubelemente (30, 32, 34, 36) jeweils entlang der Längsrichtung (L) erstrecken und entlang der Längsrichtung (L) relativ zueinander verlagerbar sind, und
wobei das erste Schubelement (30) gelenkig am ersten Endeffektorteil (18) gelagert ist, das zweite Schubelement (32) gelenkig am zweiten Endeffektorteil (20) gelagert ist, das dritte Schubelement (34) gelenkig an dem Verbindungselement (22) gelagert ist und das vierte Schubelement (36) gelenkig an dem Verbindungselement (22) gelagert ist.

2. Endoskopisches Instrument nach Anspruch 1, wobei das dritte Schubelement (34) gelenkig in einem Hohlraum des Verbindungselements (22) an dem Verbindungselement (22) gelagert ist und das vierte Schubelement (36) gelenkig in dem Hohlraum an dem Verbindungselement (22) gelagert ist.

3. Endoskopisches Instrument nach einem der vorhergehenden Ansprüche, wobei die gelenkige Lagerung des ersten Schubelements (30) derart ausgebildet ist, dass das erste Endeffektorteil (18) relativ zu dem ersten Schubelement (30) parallel zu einer Schwenkebene (50) verlagerbar ist und um eine Querachse (54) des Verbindungselements (22) verschwenkt werden kann, wobei die Schwenkebene (50) eine gedachte Ebene ist, die durch die Längsrichtung (L) und die Querrichtung (Q) aufgespannt ist.

4. Endoskopisches Instrument nach einem der vorhergehenden Ansprüche, wobei die gelenkige Lagerung des dritten Schubelements (34) derart ausgebildet ist, dass das dritte Schubelement (34) und das Verbindungselement (22) um eine Schwenkachse (56) relativ zu einander verschwenkbar sind.

5. Endoskopisches Instrument nach einem der vorhergehenden Ansprüche, wobei die gelenkige Lagerung des dritten Schubelements (34) derart ausgebildet ist, dass ein Vorsprung am dritten Schubelement (34) in eine Ausnehmung (60; 62) des Verbindungselements (22) greift oder dass ein Vorsprung am Verbindungselement in eine Ausnehmung (64) des dritten Schubelements (34) greift oder ein dritter Bolzen in zwei Ausnehmungen (60; 62) des Verbindungselements (22) eingesetzt ist und dazwischen durch eine Ausnehmung (64) am dritten Schubelement (34) geführt ist.

6. Endoskopisches Instrument nach einem der vorhergehenden Ansprüche, wobei eine gedachte Verlängerung einer ersten Hochachse (74), um die das erste Endeffektorteil (18) relativ zum ersten Schubelement (30) verschwenkbar ist, bezogen auf die Querrichtung (Q) zwischen Anlenkpunkten (42, 44) des dritten und vierten Schubelements (34, 36) an dem Verbindungselement (22) verläuft, insbesondere in der Mitte zwischen den Anlenkpunkten (42, 44).

7. Endoskopisches Instrument nach einem der vorhergehenden Ansprüche, wobei eine erste Querachse (76), um die das erste Endeffektorteil (18) relativ zum ersten Schubelement (30) verschwenkbar ist, zumindest in etwa parallel zur Querrichtung (Q) ist.

8. Endoskopisches Instrument nach einem der vorhergehenden Ansprüche, wobei das erste Schubelement (30) mittels eines ersten Gelenks (44) gelenkig am ersten Endeffektorteil (18) gelagert ist und/oder das zweite Schubelement (32) mittels eines zweiten Gelenks (46) gelenkig am zweiten Endeffektorteil (20) gelagert ist und wobei das erste Gelenk (44) eine erste Gelenkhülse (72) aufweist, die drehbar in eine Ausnehmung (73) an dem ersten Endeffektorteil (18) eingesetzt ist.

9. Endoskopisches Instrument nach Anspruch 8, wobei das erste Schubelement (30) formschlüssig mit der ersten Gelenkhülse (72) verbunden ist.

10. Endoskopisches Instrument nach Anspruch 8 oder 9, wobei die erste Gelenkhülse (72) nur um eine erste Hochachse (74) drehbar ist.

11. Endoskopisches Instrument nach einem der Ansprüche 8 bis 10, wobei das erste Schubelement (30) relativ zur ersten Gelenkhülse (72) nur in einer gedachten Ebene verlagerbar ist, die durch die Längsrichtung (L) und eine Hochrichtung (H) aufgespannt ist.

12. Endoskopisches Instrument nach einem der vorhergehenden Ansprüche, wobei alle Schubelemente (30, 32, 34, 36) parallel zueinander angeordnet sind.

13. Endoskopisches Instrument nach einem der vorhergehenden Ansprüche, wobei die Schubelemente (30, 32, 34, 36) als Schubstangen ausgestaltet sind, insbesondere vollständig geradlinig ausgestaltet sind.

14. Endoskopisches Instrument nach einem der vorhergehenden Ansprüche, wobei das erste Endeffektorteil (18) als erstes Greifzangenteil und das zweite Endeffektorteil (20) als zweites Greifzangenteil ausgebildet ist oder wobei das erste Endeffektorteil (18) als erstes Scherenteil und das zweite Endeffektorteil (20) als zweites Scherenteil ausgebildet ist.

## Claims

1. An endoscopic instrument (10) with
- a proximal end (12),
- a distal end (14),
- a longitudinal direction (L) along which the endoscopic instrument (10) extends from the proximal end (12) to the distal end (14),
- an end effector (16) at the distal end (14) comprising a first end effector portion (18) and a second end effector portion (20),
- a connecting element (22) at the distal end (14) at which the first and second end effector portions (18, 20) are arranged and which extends along a transverse direction (Q),
- a first thrust element (30),
- a second thrust element (32),
- a third thrust element (34), and
- a fourth thrust element (36),
wherein the thrust elements (30, 32, 34, 36) each extend along the longitudinal direction (L) and are displaceable relative to each other along the longitudinal direction (L), and
wherein the first thrust element (30) is mounted in an articulated manner on the first end effector portion (18), the second thrust element (32) is mounted in an articulated manner on the second end effector portion (20), the third thrust element (34) is mounted in an articulated manner on the connecting element (22), and the fourth thrust element (36) is mounted in an articulated manner on the connecting element (22).

2. The endoscopic instrument according to claim 1, wherein the third thrust element (34) is mounted in an articulated manner in a cavity of the connecting element (22) on the connecting element (22) and the fourth thrust element (36) is mounted in an articulated manner in the cavity on the connecting element (22).

3. The endoscopic instrument according to one of the preceding claims, wherein the articulated mounting of the first thrust element (30) is configured such that the first end effector portion (18) can be displaced relative to the first thrust element (30) parallel to a pivot plane (50) and can be pivoted about a transverse axis (54) of the connecting element (22), wherein the pivot plane (50) is an imaginary plane which is spanned by the longitudinal direction (L) and the transverse direction (Q).

4. The endoscopic instrument according to one of the preceding claims, wherein the articulated mounting of the third thrust element (34) is configured such that the third thrust element (34) and the connecting element (22) are pivotable relative to each other about a pivot axis (56).

5. The endoscopic instrument according to one of the preceding claims, wherein the articulated mounting of the third thrust element (34) is configured such that a projection on the third thrust element (34) engages in a recess (60; 62) of the connecting element (22) or that a projection on the connecting element engages in a recess (64) of the third thrust element (34) or that a third bolt is inserted in two recesses (60; 62) of the connecting element (22) and is guided therebetween through a recess (64) on the third thrust element (34).

6. The endoscopic instrument according to one of the preceding claims, wherein an imaginary extension of a first vertical axis (74), about which the first end effector portion (18) is pivotable relative to the first thrust element (30), extends relative to the transverse direction (Q) between articulation points (42, 44) of the third and fourth thrust elements (34, 36) on the connecting element (22), in particular in the center between the articulation points (42, 44).

7. The endoscopic instrument according to one of the preceding claims, wherein a first transverse axis (76), about which the first end effector portion (18) is pivotable relative to the first thrust element (30), is at least substantially parallel to the transverse direction (Q).

8. The endoscopic instrument according to one of the preceding claims, wherein the first thrust element (30) is mounted in an articulated manner on the first end effector portion (18) by means of a first joint (44) and/or the second thrust element (32) is mounted in an articulated manner on the second end effector portion (20) by means of a second joint (46), and wherein the first joint (44) comprises a first hinge sleeve (72) which is rotatably inserted into a recess (73) on the first end effector portion (18).

9. The endoscopic instrument according to claim 8, wherein the first thrust element (30) is positively connected to the first hinge sleeve (72).

10. The endoscopic instrument according to claim 8 or 9, wherein the first joint sleeve (72) is rotatable only about a first vertical axis (74).

11. The endoscopic instrument according to one of claims 8 to 10, wherein the first thrust element (30) is displaceable relative to the first joint sleeve (72) only in an imaginary plane which is spanned by the longitudinal direction (L) and a vertical direction (H).

12. The endoscopic instrument according to one of the preceding claims, wherein all thrust elements (30, 32, 34, 36) are arranged parallel to one another.

13. The endoscopic instrument according to one of the preceding claims, the thrust elements (30, 32, 34, 36) being configured as push rods, in particular being completely linear.

14. The endoscopic instrument according to one of the preceding claims, the first end effector portion (18) being configured as a first gripper part and the second end effector portion (20) being configured as a second gripper part, or the first end effector portion (18) being configured as a first scissor part and the second end effector portion (20) being configured as a second scissor part.

## Revendications

1. Instrument endoscopique (10) avec
- une extrémité proximale (12),
- une extrémité distale (14),
- une direction longitudinale (L) le long de laquelle l'instrument endoscopique (10) s'étend de l'extrémité proximale (12) à l'extrémité distale (14),
- un effecteur (16) à l'extrémité distale (14) comprenant une première partie effectrice (18) et une seconde partie effectrice (20),
- un élément de connexion (22) à l'extrémité distale (14) auquel les première et seconde parties effectrices (18, 20) sont supportées et qui s'étend dans une direction transversale (Q),
- un premier élément de poussée (30),
- un deuxième élément de poussée (32),
- un troisième élément de poussée (34), et
- un quatrième élément de poussée (36),
les éléments de poussée (30, 32, 34, 36) s'étendant chacun dans la direction longitudinale (L) et pouvant être déplacés l'un par rapport à l'autre dans la direction longitudinale (L), et
le premier élément de poussée (30) est monté de manière articulée sur la première partie d'effecteur (18), le deuxième élément de poussée (32) est monté de manière articulée sur la deuxième partie d'effecteur (20), le troisième élément de poussée (34) est monté de manière articulée sur l'élément de connexion (22) et le quatrième élément de poussée (36) est monté de manière articulée sur l'élément de connexion (22).

2. Instrument endoscopique selon la revendication 1, dans lequel le troisième élément de poussée (34) est monté de manière articulée dans une cavité de l'élément de connexion (22) sur l'élément de connexion (22) et le quatrième élément de poussée (36) est monté de manière articulée dans la cavité sur l'élément de connexion (22).

3. Instrument endoscopique selon l'une des revendications précédentes, dans lequel le montage articulé du premier élément de poussée (30) est conçu de telle sorte que la première partie d'effecteur (18) peut être déplacée relativement au premier élément de poussée (30) parallèlement à un plan de pivotement (50) et peut pivoter autour d'un axe transversal (54) de l'élément de connexion (22), le plan de pivotement (50) étant un plan imaginaire qui est défini par le sens longitudinal (L) et le sens transversal (Q).

4. Instrument endoscopique selon l'une des revendications précédentes, dans lequel le montage articulé du troisième élément de poussée (34) est formé de telle sorte que le troisième élément de poussée (34) et l'élément de connexion (22) peuvent pivoter l'un relativement à l'autre autour d'un axe de pivotement (56).

5. Instrument endoscopique selon l'une des revendications précédentes, dans lequel le montage articulé du troisième élément de poussée (34) est conçu de telle sorte qu'une saillie sur le troisième élément de poussée (34) s'engage dans un évidement (60; 62) de l'élément de connexion (22) ou qu'une saillie sur l'élément de connexion s'engage dans un évidement (64) du troisième élément de poussée (34) ou qu'un troisième boulon est inséré dans deux évidements (60; 62) de l'élément de connexion (22) et est guidé entre ceux-ci par un évidement (64) du troisième élément de poussée (34).

6. Instrument endoscopique selon l'une des revendications précédentes, dans lequel un prolongement imaginaire d'un premier axe vertical (74), autour duquel la première partie d'effecteur (18) peut pivoter relativement au premier élément de poussée (30), s'étend par rapport au sens transversal (Q) entre des points d'articulation (42, 44) des troisième et quatrième éléments de poussée (34, 36) de l'élément de connexion (22), en particulier dans le centre entre les points d'articulation (42, 44).

7. Instrument endoscopique selon l'une des revendications précédentes, dans lequel un premier axe transversal (76) autour duquel la première partie d'effecteur (18) peut pivoter relativement au premier élément de poussée (30) est au moins approximativement parallèle au sens transversal (Q).

8. Instrument endoscopique selon l'une des revendications précédentes, dans lequel le premier élément de poussée (30) est monté de manière articulée sur la première partie d'effecteur (18) au moyen d'une première articulation (44) et/ou le deuxième élément de poussée (32) est monté de manière articulée sur la deuxième partie d'effecteur (20) au moyen d'une deuxième articulation (46) et dans lequel la première articulation (44) comprend un premier manchon articulé (72) inséré rotatif dans une cavité (73) sur la première partie d'effecteur (18).

9. Instrument endoscopique selon la revendication 8, le premier élément de poussée (30) étant relié positivement au premier manchon articulé (72).

10. Instrument endoscopique selon la revendication 8 ou 9, dans lequel le premier manchon articulé (72) peut tourner uniquement autour d'un premier axe vertical (74).

11. Instrument endoscopique selon l'une quelconque des revendications 8 à 10, dans lequel le premier élément de poussée (30) ne peut être déplacé relativement au premier manchon articulé (72) que dans un plan imaginaire qui est défini par la direction longitudinale (L) et une direction verticale (H).

12. Instrument endoscopique selon l'une des revendications précédentes, dans lequel tous les éléments de poussée (30, 32, 34, 36) sont disposés parallèlement les uns aux autres.

13. Instrument endoscopique selon l'une des revendications précédentes, dans lequel les éléments de poussée (30, 32, 34, 36) sont réalisés sous forme de tiges de poussée, en particulier de manière complètement linéaire.

14. Instrument endoscopique selon l'une des revendications précédentes, dans lequel la première partie d'effecteur (18) est formée en tant que première partie de pince et la deuxième partie d'effecteur (20) est formée en tant que deuxième partie de pince, ou dans lequel la première partie d'effecteur (18) est formée en tant que première partie ciseaux et la deuxième partie effecteur (20) est formée en tant que deuxième partie ciseaux.
